# EUROPEAN PATENT SPECIFICATION

(11) **EP 4 338 232 B1**
(45) Date of publication and mention of the grant of the patent: **25.03.2026**
(21) Application number: 21772881.5
(22) Date of filing: 20.08.2021
(51) Int. Cl.: H01Q 1/27, G04R 60/06, H01Q 5/378, A61B 5/28, A61B 5/00, G04G 17/04

(54) **WEARABLE COMPUTING DEVICE HAVING A MULTI-BAND SLOT ANTENNA AND A GROUNDED PARASITIC ELEMENT**
TRAGBARE COMPUTERVORRICHTUNG MIT MEHRBANDSCHLITZANTENNE UND GEERDETEM PARASITÄREM ELEMENT
DISPOSITIF INFORMATIQUE PORTABLE AYANT UNE ANTENNE À FENTES MULTIBANDE ET UN ÉLÉMENT PARASITE MIS À LA TERRE

(43) Date of publication of application: 20.03.2024
(73) Proprietor: Fitbit LLC, San Francisco, CA 94105 (US)
(72) Inventor: VERMA, Ritu, San Francisco, California 94105 (US); ZHENG, Tianji, San Francisco, California 94105 (US); LI, Kevin, San Francisco, California 94105 (US)
(74) Representative: Maikowski & Ninnemann Patentanwälte Partnerschaft mbB
(86) International application number: PCT/US2021/046930
(87) International publication number: WO 2023/022732

(56) References cited:
- US-A1- 2017 048 991
- US-A1- 2019 109 367
- US-A1- 2020 015 701
- US-A1- 2020 192 424

## Description

### FIELD

The present disclosure relates generally to wearable computing devices. More particularly, the present disclosure relates to a wearable computing device having a grounded parasitic element to improve performance (e.g., radiation efficiency) of a slot antenna defined by a gap between a printed circuit board of the wearable computing device and a conductive housing of the wearable computing device.

### BACKGROUND

Modern electronic devices frequently include one or more radio-frequency (RF) antennas to facilitate wireless communication with other electronic devices. For example, in wearable computing devices, the RF antennas must fit within a restricted space while still providing desirable emission and reception characteristics. Furthermore, it can be desirable for these wearable computing devices to support communication over multiple frequency bands. US 2017/048991 A1, US 2019/109367 A1 and US 2020/192424 A1 disclose wearable computing devices comprising parasitic elements. US 2020/015701 A1 discloses a wearable computing device wherein an electrocardiogram electrode is further used as antenna element.

### SUMMARY

Aspects and advantages of embodiments of the present disclosure will be set forth in part in the following description, or can be learned from the description, or can be learned through practice of the embodiments.

In one aspect, a wearable computing device is provided. The wearable computing device includes a printed circuit board and a conductive housing. The wearable computing device further includes a slot antenna defined by a gap between the printed circuit board and the conductive housing. The slot antenna is operable at a plurality of different frequency bands. The plurality of different frequency bands include one or more global position system frequency bands. The wearable computing device includes a parasitic element. The parasitic element is electrically grounded to the printed circuit board at a plurality of different locations. The parasitic element is an electrocardiogram electrode (ECG) electrode.

In some implementations, the parasitic element is direct current grounded to the printed circuit board at a first location thereon. In addition, the parasitic element is direct current grounded to the printed circuit board at a second location thereon. In some implementations, the first location and the second location are spaced apart from one another such that one or more magnets disposed on the printed circuit board are positioned between the first location and the second location. In some implementations, the parasitic element is radio frequency grounded to the printed circuit board via one or more bypass capacitors.

In some implementations, a width of the gap between the printed circuit board and the conductive housing ranges from about 0.5 millimeters to about 10 millimeters. In some implementations, a perimeter of the printed circuit board includes a ground keep-out region. Furthermore, in some implementations, a width of the slot antenna spans the width of the gap and a width of the ground keep-out region. In some implementations, the width of the slot antenna ranges from about 0.5 millimeters to about 10 millimeters.

In some implementations, the slot antenna induces one or more electrical currents on the parasitic element when the slot antenna operates at the one or more global positioning system frequency bands.

In some implementations, the slot antenna includes a first grounding contact and a second grounding contact. The first grounding contact is coupled between the printed circuit board and the conductive housing at a first location. The second grounding contact is coupled between the printed circuit board and the conductive housing at a second location that is different than the first location.

In some implementations, the one or more global positioning system frequency bands include a first global positioning system frequency band ranging from about 1164 Megahertz to about 1189 Megahertz, a second global positioning system frequency band ranging from about 1563 Megahertz to about 1587 Megahertz, and a third global positioning system frequency band ranging from about 1215 Megahertz to about 1240 Megahertz.

In some implementations, a radiation efficiency of the slot antenna at the one or more global positioning system frequency bands is increased by at least 2 decibels due, at least in part, to the parasitic element being radio frequency grounded to the printed circuit board via the one or more bypass capacitors.

In some implementations, the electrocardiogram electrode is radio frequency grounded to the printed circuit board at a first location thereon via a first spring clip. Additionally, the electrocardiogram electrode is radio frequency grounded to the printed circuit board at a second location thereon via a second spring clip. In some implementations, the first location and the second location are spaced apart from one another on the printed circuit board such that one or more magnets disposed on the printed circuit board are positioned between the first location and the second location.

In some implementations, an electrodermal activity electrode is electrically coupled to the printed circuit board.

These and other features, aspects, and advantages of various embodiments of the present disclosure will become better understood with reference to the following description and appended claims. The accompanying drawings, which are incorporated in and constitute a part of this specification, illustrate example embodiments of the present disclosure and, together with the description, serve to explain the related principles.

### BRIEF DESCRIPTION OF THE DRAWINGS

Detailed discussion of embodiments directed to one of ordinary skill in the art is set forth in the specification, which makes reference to the appended figures, in which:
FIG. 1 depicts a wearable computing device according to some implementations of the present disclosure.
FIG. 2 depicts a conductive housing of a wearable computing device according to some implementations of the present disclosure.
FIG. 3 depicts a side view of a wearable computing device with a bottom cover of a housing assembly of the wearable computing device removed according to some implementations of the present disclosure.
FIG. 4 depicts the side view of a wearable computing device with a housing assembly of the wearable computing device removed according to some implementations of the present disclosure.
FIG. 5 depicts a printed circuit board positioned within a conductive housing of a wearable computing device according to some implementations of the present disclosure.
FIG. 6 depicts a printed circuit board of a wearable computing device according to some implementations of the present disclosure.
FIG. 7 depicts a slot antenna defined by a gap between a conductive housing and a printed circuit board of a wearable computing device according to some implementations of the present disclosure.
FIG. 8 depicts a parasitic element grounded to a printed circuit board of a wearable computing device according to some implementations of the present disclosure.
FIG. 9 depicts a parasitic element radio frequency grounded to a printed circuit board of a wearable computing device according to some implementations of the present disclosure.
FIG. 10 depicts a schematic of a layout of a printed circuit board of a wearable computing device according to some implementations of the present disclosure.

### DETAILED DESCRIPTION

Reference now will be made in detail to embodiments of the invention, one or more examples of which are illustrated in the drawings. Each example is provided by way of explanation of the invention, not limitation of the invention. In fact, it will be apparent to those skilled in the art that various modifications and variations can be made in the present invention without departing from the scope of the invention as defined by the appended claims. For instance, features illustrated or described as part of one embodiment can be used with another embodiment to yield a still further embodiment. Thus, it is intended that the present invention covers such modifications and variations as come within the scope of the appended claims.

Example aspects of the present disclosure are directed to a wearable computing device that can be worn, for instance, on a user's wrist or other location on the user's body. The wearable computing device can include a slot antenna defined by a gap (e.g., about a 0.1 mm gap to about a 10 mm gap) between a conductive housing (e.g., metal housing) and a printed circuit board. The slot antenna can operate at a plurality of different frequency bands. For example, the slot antenna can operate at one or more global navigation satellite system (e.g., global positioning system (GPS), GLONASS, Galileo, etc.) frequency bands. For instance, the one or more global navigation satellite system frequency bands can include one or more GPS frequency bands (e.g., 1164 MHz to 1189 MHz, 1563 MHz to 1587 MHz, 1215 MHz to 1240 MHz,). However, since the wearable computing device must be compact enough to be worn, for instance, on the user's wrist, the dimensions (e.g., length) of the slot antenna are limited, which can, in some circumstances, impact the performance (e.g., radiation efficiency) of the slot antenna at the one or more GPS frequency bands.

One aspect of the present disclosure is directed to a wearable computing device having a parasitic element. The parasitic element can be electrically grounded to the printed circuit board. For instance, the parasitic element can be electrically grounded to a ground plane of the printed circuit board. It should be understood that the parasitic element can include any element that introduces a parasitic resonance. For example, the parasitic element can include a metal element (e.g., decorative element). Examples of a wearable computing device having a parasitic element in the form of an electrocardiogram (ECG) electrode are discussed further below.

The parasitic element can be direct current (DC) grounded to the printed circuit board at a first location thereon. Additionally, the parasitic element can be DC grounded to the printed circuit board at a second location thereon. The first location and the second location can be spaced apart from one another along the printed circuit board. Furthermore the printed circuit board can include a first fastener (e.g., spring clip) at the first location and a second fastener (e.g., spring clip) at the second location to couple the parasitic element to the printed circuit board at the first location and the second location, respectively. For example, a first pole of the parasitic element and/or a second pole of the parasitic element can be mechanically coupled and/or electrically coupled to the printed circuit board via the first fastener and the second fastener, respectively.

The parasitic element can be radio frequency (RF) grounded to the printed circuit board at multiple different locations. In this manner, the slot antenna induces one or more electrical currents on the parasitic element when operating at the one or more GPS frequency bands, which can improve performance (e.g., radiation efficiency) of the slot antenna at the one or more GPS frequency bands. For example, the parasitic element being RF grounded to the printed circuit board at multiple locations can, in some instances, improve the radiation efficiency of the slot antenna at the one or more GPS frequency bands by at least about 2 decibels. As used herein, the use of the term "about" in conjunction with a numerical value refers to within about 20% of the stated numerical value.

In some implementations, the parasitic element can be radio frequency (RF) grounded to the printed circuit board via one or more bypass capacitors. In this manner, the parasitic element can be electrically isolated at DC or low-frequencies (e.g., non-RF frequencies). For instance, in some implementations, the parasitic element can be RF grounded to the printed circuit board at the first location thereon via a first bypass capacitor. Alternatively, or additionally, the parasitic element can be RF grounded to the printed circuit board at the second location thereon via a second bypass capacitor.

Another aspect of the present disclosure is directed to a wearable computing device having an ECG electrode. The ECG electrode can be RF grounded to the printed circuit board at a plurality of different locations. Furthermore, since the ECG electrode is RF grounded, the slot antenna can induce one or more electrical currents on the ECG electrode when the slot antenna is operating at the one or more GPS frequency bands. In this manner, the ECG electrode being RF grounded to the printed circuit board at multiple locations can improve performance (e.g., radiation efficiency) of the slot antenna at the one or more GPS frequency bands. For example, the radiation efficiency of the slot antenna at the one or more GPS frequency bands can be improved by at least 2 decibels. Furthermore, since the ECG electrode can double as the parasitic element, performance of the slot antenna at the one or more GPS frequency bands can be improved without requiring a separate parasitic element.

The ECG can be RF grounded to the printed circuit board via one or more bypass capacitors. For instance, the ECG electrode can be RF grounded via a first bypass capacitor coupled between the printed circuit board and a first pole of the ECG electrode. Additionally, the ECG electrode can be RF grounded via a second bypass capacitor coupled between the printed circuit board and a second pole of the ECG electrode. Furthermore, in some instances, the first bypass capacitor and/or the second bypass capacitor can be mechanically and electrically coupled to the printed circuit board via the first fastener and the second fastener, respectively.

A wearable computing device according to example aspects of the present disclosure can provide numerous technical effects and benefits. For instance, RF grounding a parasitic element or ECG electrode to the printed circuit board at multiple locations can improve performance (e.g., radiation efficiency) of the slot antenna at the one or more GPS frequency bands. Furthermore, RF grounding the ECG electrode to the printed circuit can allow the ECG electrode to double as an ECG sensor and a parasitic element. In this manner, performance (e.g., radiation efficiency) of the slot antenna can be improved without requiring a separate parasitic element.

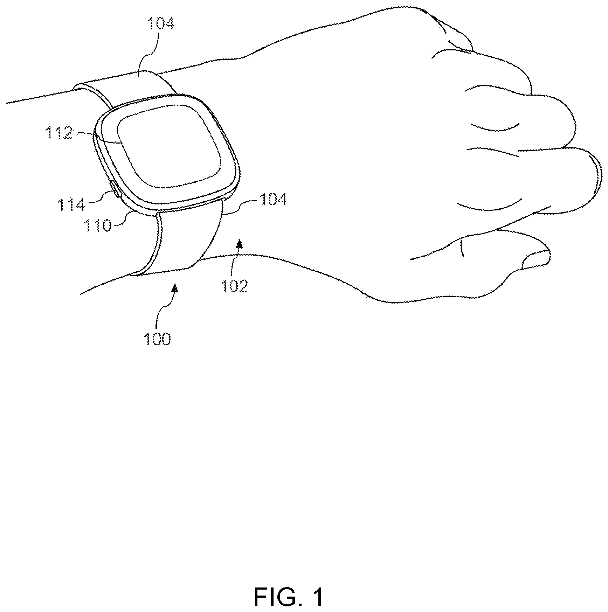

Referring now to the FIGS., FIG. 1 depicts a wearable computing device 100 according to some implementations of the present disclosure. As shown, the wearable computing device 100 can be worn, for instance, on an arm 102 (e.g., wrist) of a user. For instance, the wearable computing device 100 can include a band 104 and a housing assembly 110. The housing assembly 110 can be coupled to the band 104. In this manner, the band 104 can be fastened to the arm 102 of the user to secure the housing assembly 110 to the arm 102 of the user.

In some implementations, the wearable computing device 100 can include a display 112 that can display content (e.g., time, date, etc.) to the user. In some implementations, the display 112 can include an interactive display (e.g., touchscreen or touch-free). In such implementations, the user can interact with the wearable computing device 100 via the display 112 to control operation of the wearable computing device 100. Alternatively, or additionally, the wearable computing device 100 can include one or more input devices 114 that can be manipulated by the user to interact with the wearable computing device 100. For instance, the one or more input devices 114 can include a mechanical button that can be manipulated (e.g., pressed) to interact with the wearable computing device 100. In some implementations, the one or more input devices 114 can be manipulated to control operation of a backlight (not shown) associated with the display 112. It should be understood that the one or more input device 114 can be configured to allow the user to interact with the wearable computing device 100 in any suitable manner. For instance, in some implementations, the one or more input device 114 can be manipulated by the user to navigate through one or more menus on the display 112.

In some implementations, the wearable computing device 100 can be designed to be worn (e.g., continuously) by the user. When worn, the wearable computing device 100 can gather data regarding activities performed by the user, or regarding the user's physiological state. Such data may include data representative of the ambient environment around the user or the user's interaction with the environment. For example, the data can include motion data regarding the user's movements, ambient light, ambient noise, air quality, etc., and/or physiological data obtained by measuring various physiological characteristics of the user, such as heart rate, perspiration levels, and the like.

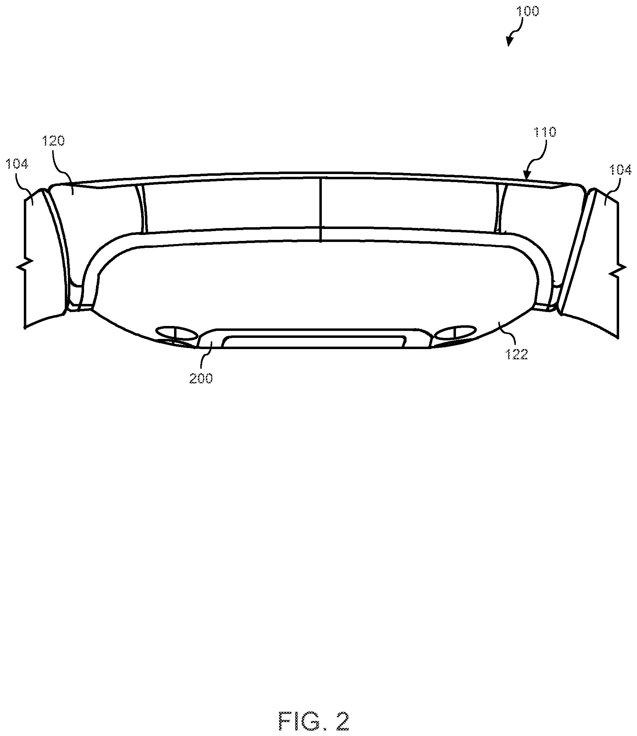

Referring now to FIG. 2, a side view of the housing assembly 110 of the wearable computing device 100 is provided according to some implementations of the present disclosure. As shown, the housing assembly 110 can include a conductive housing 120. The conductive housing 120 can be attached to the band 104 that is used to secure the housing assembly 110 to the arm 102 (FIG. 1) of the user. The housing assembly 110 can include a cover 122 coupled to the conductive housing 120. In some implementations, the cover 122 can be coupled to the bottom of the conductive housing 120. In this manner, the cover 122 can contact (e.g., touch) the arm 102 (FIG. 1) of the user when the housing assembly 110 is secured to the arm 102 of the user via the band 104.

The conductive housing 120 can include any suitable conductive material. For instance, in some implementations, the conductive housing 120 can include a metal housing. The cover 122 can include an insulating material. For instance, in some implementations, the cover 122 can include a plastic cover.

In some implementations, the wearable computing device 100 can include an electrocardiogram (ECG) electrode 200. As shown, the ECG electrode 200 can be positioned within an opening (e.g., cutout) defined by the cover 122. In this manner, the ECG electrode 200 can contact (e.g., touch) the arm 102 (FIG. 1) of the user when the housing assembly 110 is secured to the arm 102 of the user via the band 104. When the ECG electrode 200 contacts the arm 102 of the user, the ECG electrode 200 can be electrically connected to the arm 102 (e.g., wrist) of the user. Furthermore, it should be understood that the wearable computing device 100 can determine one or more health metrics (e.g., heart rate) of the user based, at least in part, on data obtained via the ECG electrode 200 when the ECG electrode 200 is electrically connected to the arm 102 (e.g., wrist) of the user.

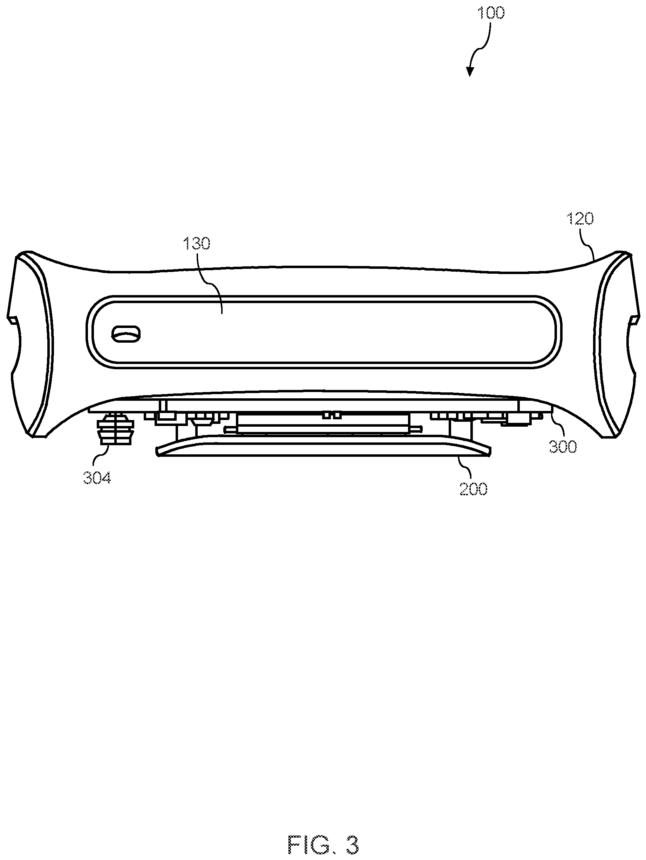

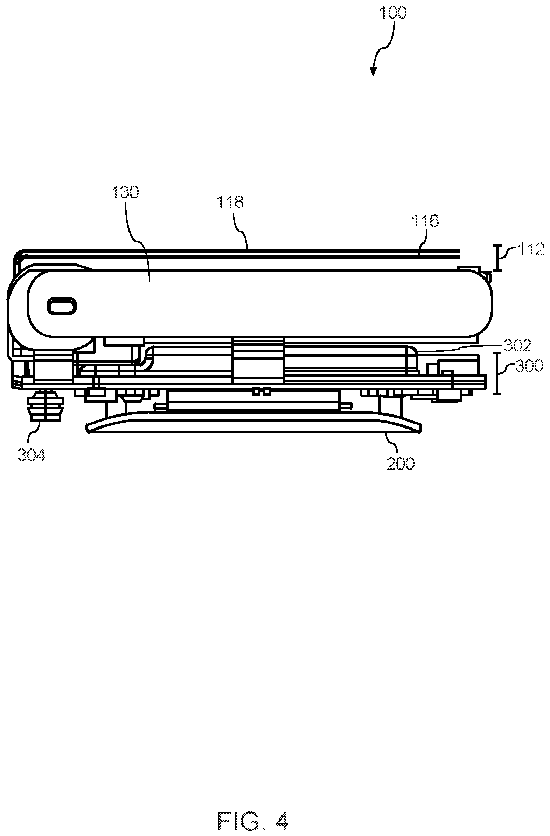

Referring now to FIGS. 3 and 4, a side view of the wearable computing device 100 is provided according to some implementations. FIG. 3 depicts a side view of the wearable computing device 100 with the cover 122 (FIG. 2) removed. FIG. 4 depicts a side view of the wearable computing device 100 without the housing assembly 110 (FIG. 2). As shown, the display 112 can, in some implementations, include a display ITO coating 116 and a touch ITO coating 118.

The wearable computing device 100 can include a printed circuit board 300 disposed within the housing assembly 110 (FIG. 2). For instance, in some implementations, a first portion of the printed circuit board 300 can be positioned within the conductive housing 120 and a second portion of the printed circuit board 300 can be positioned within the cover 122. The printed circuit board 300 can include a plurality of electronic components (not shown) disposed thereon. In some implementations, the printed circuit board 300 can include a shielding can 302 covering at least a portion of the printed circuit board 300. In this manner, the shielding can 302 can cover one or more electronic components of the plurality of electronic components disposed on the printed circuit board 300. Alternatively, or additionally, the printed circuit board 300 can include one or more charging pins 304. In this manner, the wearable computing device 100 can be coupled to a charging circuit (not shown) via the one or more charging pins 304 to facilitate charging of an energy storage device (e.g., battery) of the wearable computing device 100.

In some implementations, the conductive housing 120 can define an opening (e.g., cutout) for one or more sensors 130. In this manner, the one or more sensors 130 can be visible to the user. In some implementations, the one or more sensors 130 can include at least one of an electrodermal activity (EDA) electrode and an ECG electrode. In such implementations, the user can contact (e.g., touch) the one or more sensors 130 to facilitate measuring one or more health metrics (e.g., heart-rate, blood pressure, ECG, EDA etc.) of the user. It should be understood that the one or more sensors 130 can be electrically coupled to the printed circuit board 300.

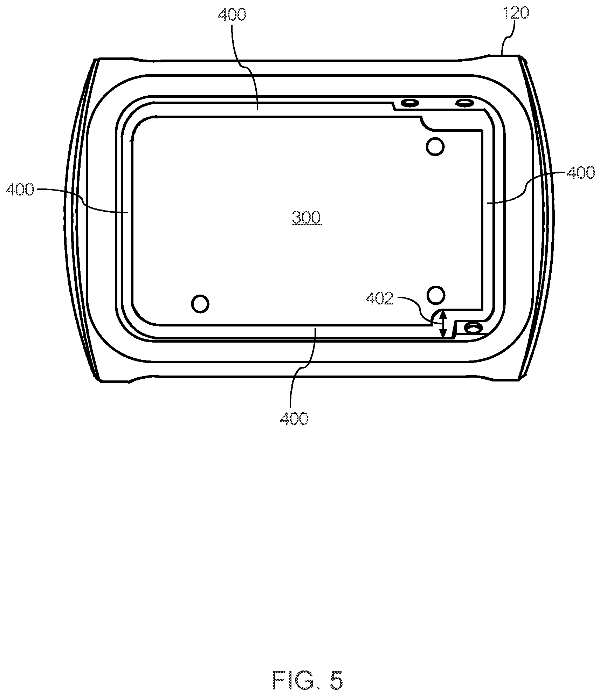

Referring now to FIG. 5, the printed circuit board 300 is positioned relative to the conductive housing 120 such that a gap 400 is defined between the conductive housing 120 and the printed circuit board 300. The gap 400 can extend around the entire perimeter of the printed circuit board 300. Stated another way, no edge of the printed circuit board 300 can contact (e.g., touch) the conductive housing 120.

In some implementations, a width 402 of the gap 400 defined between the conductive housing 120 and the printed circuit board 300 can range from about 0.5 millimeters to about 10 millimeters. In some implementations, the width 402 of the gap 400 can vary around the perimeter of the printed circuit board 300. For instance, the width 402 of the gap 400 between the conductive housing 120 and the printed circuit board 300 at a first portion of the perimeter of the printed circuit board 300 can be different (e.g., wider, narrower) than the width 402 of the gap 400 between the conductive housing 120 and the printed circuit board 300 at a second portion of the perimeter of the printed circuit board 300.

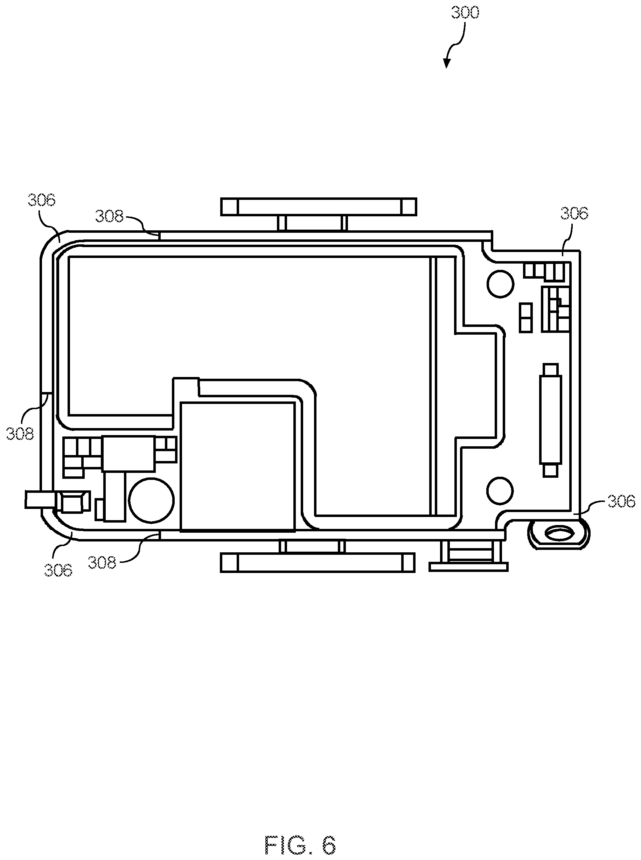

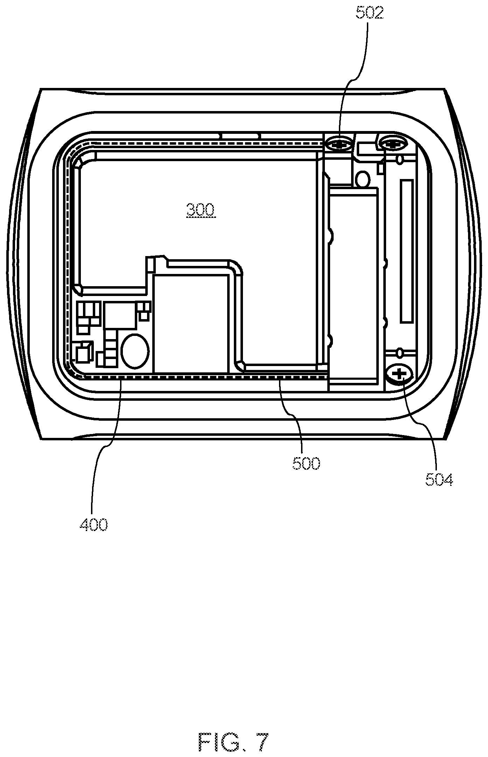

Referring now to FIGS. 6 and 7, the perimeter of the printed circuit board 300 can, in some implementations, include a copper free or ground keep-out region 306. It should be understood that the ground keep-out region 306 can include a region of the printed circuit board 300 where electronic components (e.g., resistors, capacitors, etc.) cannot be placed. In some implementations, a width 308 of the ground keep-out region 306 of the printed circuit board 300 can range from 0.1 millimeters to about 2 millimeters. As will be discussed below, the ground keep-out region 306 can act as an electrical gap.

In some implementations, a slot antenna 500 (denoted by dashed line) can be defined by the gap 400 between the conductive housing 120 and the printed circuit board 300. Furthermore, in some implementations, the slot antenna 500 can be further defined by an electrical gap that spans the width 308 of the ground keep-out region 306 of the printed circuit board 300. In such implementations, the width of the slot antenna 500 can span the width 402 (FIG. 5) of the gap 400 and the width 308 of the ground keep-out region 306 of the printed circuit board 300. For instance, in some implementations, the width of the slot antenna 500 can range from about 0.5 millimeters to about 10 millimeters.

The slot antenna 500 can be operable at a plurality of different frequency bands. For instance, the slot antenna 500 can be operable at one or more Global Navigation Satellite System (GNSS) frequency bands. In some implementations, the one or more GNSSS frequency bands can include one or more GPS frequency bands. The one or more GPS frequency bands can include at least one of a first GPS frequency band ranging from about 1164 Megahertz (MHz) to about 1189 MHz, a second GPS frequency band ranging from about 1563 MHz to about 1587 MHz, and a third GPS frequency band ranging from about 1215 MHz to about 1240 MHz. Furthermore, in addition to the one or more GPS frequency bands, the slot antenna 500 can be configured to radiate at one or more frequency bands associated with cellular communications (e.g., 4G, 5G) or wireless local area communications. It should be understood however that the slot antenna 500 can be operable at frequency bands associated with any suitable communication standard.

In some implementations, the slot antenna 500 can include a first grounding contact 502 and a second grounding contact 504. The first grounding contact 502 can be coupled between the conductive housing 120 and a first location on the perimeter (e.g., ground keep-out region 306) of the printed circuit board 300. Conversely, the second grounding contact 504 can be coupled between the conductive housing 120 and a second location on the perimeter 306 of the printed circuit board 300. In some implementations, the first location and the second location can correspond to opposing sides of the printed circuit board 300. It should be understood however that the first grounding contact 502 and the second grounding contact 504 can be coupled to the perimeter of the printed circuit board 300 at any suitable location to adjust a length of the slot antenna 500. For instance, the first grounding contact 502 and the second grounding contact 504 can be positioned closer to one another to shorten the slot antenna 500. Alternatively, the first grounding contact 502 and the second grounding contact 504 can be positioned farther apart from one another to lengthen the slot antenna 500.

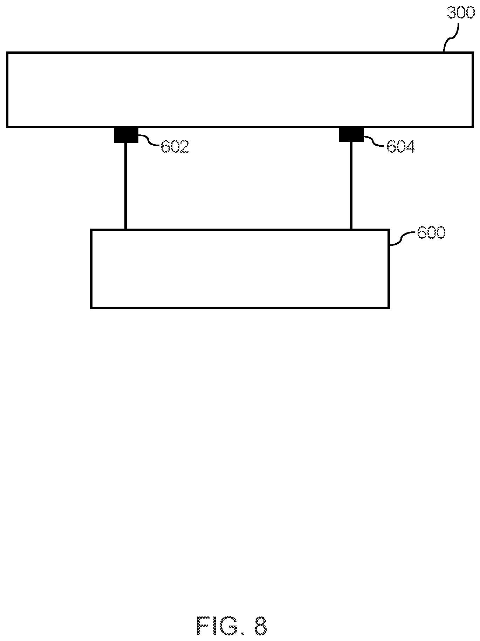

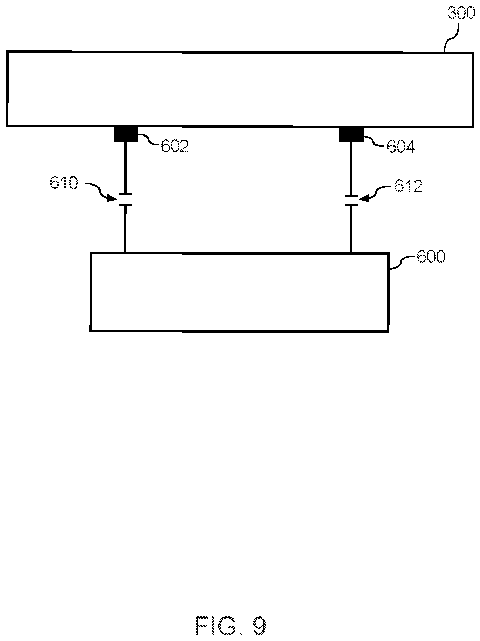

Referring now to FIGS. 8 and 9, a parasitic element 600 of a wearable computing device (e.g., wearable computing device 100 of FIG. 1) is provided according to some implementations of the present disclosure. In some implementations, the parasitic element 600 can be disposed entirely within the housing assembly 110 (FIG. 1) of the wearable computing device 100. In alternative implementations, the parasitic element 600 can be partially positioned within the housing assembly 110. For instance, a first portion of the parasitic element 600 can be positioned within the housing assembly 110, whereas a second portion of the parasitic element 600 can be positioned outside of the housing assembly 110.

The parasitic element 600 can be electrically grounded to the printed circuit board 300. For instance, the parasitic element 600 can be electrically grounded to a ground plane of the printed circuit board 300. In some implementations, the parasitic element 600 can be DC grounded to the printed circuit board 300 at multiple locations. For instance, the parasitic element 600 can be DC grounded to the printed circuit board 300 at a first location 602 thereon and a second location 604 thereon. As shown, the first location 602 and the second location 604 can be spaced apart from one another along the printed circuit board 300. It should be understood that, in some implementations, the parasitic element 60 can be DC grounded to the printed circuit board 300 at more than two locations (e.g., first location 602, second location 604) thereon.

In some implementations, the parasitic element 600 can be RF grounded to the printed circuit board 300 via one or more bypass capacitors. For instance, in some implementations the parasitic element 600 can be RF grounded to the printed circuit board 300 at the first location 602 thereon via a first bypass capacitor 610. Additionally, the parasitic element 600 can be RF grounded to the printed circuit board 300 at the second location 604 thereon via a second bypass capacitor 612. It should be understood that the bypass capacitors (e.g., first bypass capacitor 610 and second bypass capacitor 612) can leave the parasitic element 600 electrically isolated to electrical ground at direct current and low frequencies (e.g., non-RF frequencies).

The parasitic element 600 can improve performance (e.g., radiation efficiency) of the slot antenna 500 at the one or more GPS frequency bands. For instance, since the parasitic element 600 is electrically grounded (e.g., radio frequency grounded, DC grounded) to the printed circuit board 300 at multiple locations (e.g., first location 602, second location 604), the slot antenna 500 can induce one or more electrical currents on the parasitic element 600 when the slot antenna 500 is operating at the one or more GPS frequency bands. It should be understood that the slot antenna 500 inducing one or more electrical currents on the parasitic element 600 can improve performance (e.g., radiation efficiency) of the slot antenna 500 at the one or more GPS frequency bands. For instance, in some implementations, the radiation efficiency of the slot antenna 500 can increase by at least 2 decibels due, at least in part, to the parasitic element 600 being electrically grounded to the printed circuit board 300 at multiple locations (e.g., first location 602, second location 604).

In some implementations not forming part of the claimed invention, the parasitic element 600 can include a metal element that is separate from the ECG electrode 200 (FIG. 3) of the wearable computing device 100 (FIG. 3). According to the invention, the parasitic element 600 includes the ECG electrode 200 discussed above with reference to FIGS. 2-4. Additionally, in some implementations, the ECG electrode 200 can be RF grounded to the printed circuit board 300 via one or more bypass capacitors (e.g., first bypass capacitor 610, second bypass capacitor 612). It should be appreciated that RF grounding the ECG electrode 200 to the printed circuit board 300 at multiple locations can allow the performance of the slot antenna 500 (FIG. 7) at the one or more GPS frequency bands to be improved without requiring a separate parasitic element 600.

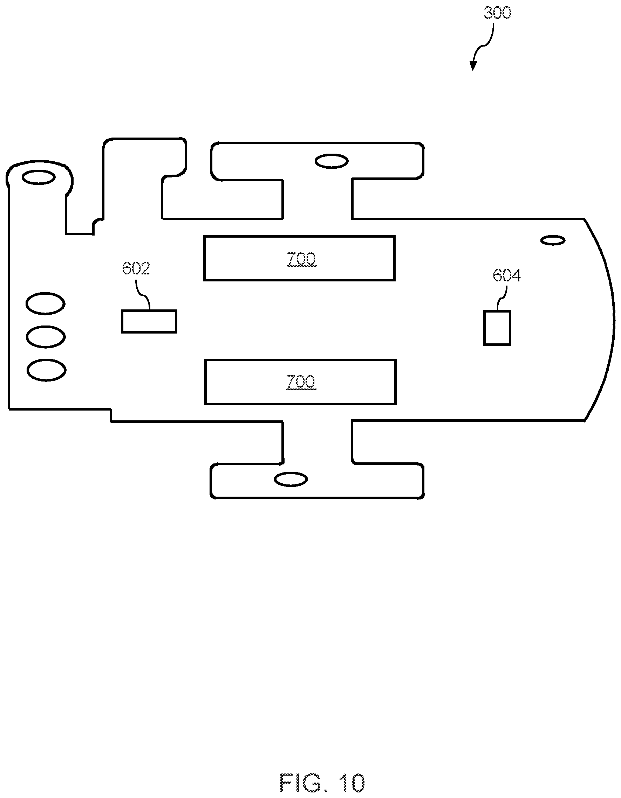

Referring now to FIG. 10, a schematic of a layout of the printed circuit board 300 is provided according to some implementations of the present disclosure. As shown, the first location 602 and the second location 604 can be spaced apart from one another on the printed circuit board 300 such that one or more magnets 700 are positioned between the first location 602 and the second location 604. For instance, in some implementations, the one or more magnets 700 can include a first magnet and a second magnet. In alternative implementations, more or fewer magnets are positioned between the first location and the second location.

In some implementations, the printed circuit board 300 can include a first fastener (e.g., spring clip) at the first location 602 thereon and a second fastener (e.g., spring clip) at the second location thereon. In this manner, the parasitic element 600 can be mechanically coupled to the printed circuit board 300 at the first location 602 and the second location 604 via the first fastener and the second fastener, respectively.

While the present subject matter has been described in detail with respect to various specific example embodiments thereof. each example is provided by way of explanation, not limitation of the disclosure. Those skilled in the art, upon attaining an understanding of the foregoing, can readily produce alterations to, variations of, and equivalents to such embodiments. Accordingly, the subject disclosure does not preclude inclusion of such modifications, variations and/or additions to the present subject matter as would be readily apparent to one of ordinary skill in the art. For instance, features illustrated or described as part of one embodiment can be used with another embodiment to yield a still further embodiment. Thus, it is intended that the present disclosure cover such alterations, variations, and equivalents.

## Claims

1. A wearable computing device (100) comprising:
a printed circuit board (300);
a conductive housing (120);
a slot antenna (500) defined by a gap (400) between the printed circuit board and the conductive housing, the slot antenna operable at a plurality of different frequency bands, the plurality of different frequency bands comprising one or more global positioning system, GPS, frequency bands; and
a parasitic element (600),
the parasitic element being electrically grounded to the printed circuit board at a plurality of different locations,
the wearable computing device being **characterized in that**
the parasitic element is an electrocardiogram electrode, ECG, electrode (200).

2. The wearable computing device of claim 1, wherein the parasitic element is radio frequency, RF, grounded to the printed circuit board via one or more bypass capacitors (610, 612).

3. The wearable computing device of claim 1, wherein:
the parasitic element is direct current, DC, grounded to the printed circuit board at a first location (602) thereon; and
the parasitic element is DC grounded to the printed circuit board at a second location (604) thereon.

4. The wearable computing device of claim 3, wherein the first location and the second location are spaced apart from one another on the printed circuit board such that one or more magnets (700) disposed on the printed circuit board are positioned between the first location and the second location.

5. The wearable computing device of any of the preceding claims, wherein a width of the gap between the printed circuit board and the conductive housing ranges from about 0.5 millimeters to about 10 millimeters.

6. The wearable computing device of claim 1, wherein a perimeter of the printed circuit board comprises a ground keep-out regior (306).

7. The wearable computing device of claim 6, wherein a width of the slot antenna spans the width of the gap and a width of the ground keep-out region.

8. The wearable computing device of claim 7, wherein the width of the slot antenna ranges from about 0.5 millimeters to about 10 millimeters.

9. The wearable computing device of any of the preceding claims, configured so that when the slot antenna operates at the one or more GPS frequency bands, the slot antenna induces one or more electrical currents on the parasitic element.

10. The wearable computing device of any of the preceding claims, wherein the slot antenna comprises a first grounding contact (502) and a second grounding contact (504), the first grounding contact coupled between the printed circuit board and the conductive housing at a first location, the second grounding contact coupled between the printed circuit board and the conductive housing at a second location that is different than the first location.

11. The wearable computing device of any of the preceding claims, wherein the one or more GPS frequency bands comprise:
a first GPS frequency band ranging from about 1164 MHz to about 1189 MHz
a second GPS frequency band ranging from about 1563 MHz to about 1587 MHz; and
a third GPS frequency band ranging from about 1215 MHz to about 1240 MHz.

12. The wearable computing device of claim 2, configured so that a radiation efficiency of the slot antenna at the one or more GPS frequency bands is increased by at least 2 decibels due, at least in part, to the parasitic element being RF grounded to the printed circuit board via the one or more bypass capacitors.

13. The wearable computing device of any of the preceding claims, wherein:
the parasitic element is RF grounded to the printed circuit board at a first location thereon via a first a first spring clip; and
the parasitic element is RF grounded to the printed circuit board at a second location thereon via a second spring clip.

14. The wearable computing device of any of the preceding claims, further comprising:
an electrodermal activity electrode electrically coupled to the printed circuit board.

## Patentansprüche

1. Tragbare Rechenvorrichtung (100), umfassend:
eine Leiterplatte (300);
ein leitfähiges Gehäuse (120);
eine Schlitzantenne (500), die durch einen Spalt (400) zwischen der Leiterplatte und dem leitfähigen Gehäuse definiert ist, wobei die Schlitzantenne in einer Vielzahl von verschiedenen Frequenzbändern betreibbar ist, wobei die Vielzahl von verschiedenen Frequenzbändern ein oder mehrere Frequenzbänder eines globalen Positionsbestimmungssystems, GPS, umfasst; und
ein parasitäres Element (600), wobei das parasitäre Element an einer Vielzahl von verschiedenen Stellen mit der Leiterplatte elektrisch an Masse gelegt ist, wobei die tragbare Rechenvorrichtung **dadurch gekennzeichnet ist, dass** das parasitäre Element eine Elektrokardiogramm-Elektrode, EKG-Elektrode, (200) ist.

2. Tragbare Rechenvorrichtung nach Anspruch 1, wobei das parasitäre Element über einen oder mehrere Überbrückungskondensatoren (610, 612) für Hochfrequenz, HF, mit der Leiterplatte an Masse gelegt ist.

3. Tragbare Rechenvorrichtung nach Anspruch 1, wobei:
das parasitäre Element an einer ersten Stelle (602) darauf für Gleichstrom, DC, mit der Leiterplatte an Masse gelegt ist; und das parasitäre Element an einer zweiten Stelle (604) darauf für DC mit der Leiterplatte an Masse gelegt ist.

4. Tragbare Rechenvorrichtung nach Anspruch 3, wobei die erste Stelle und die zweite Stelle auf der Leiterplatte so voneinander beabstandet sind, dass ein oder mehrere auf der Leiterplatte angeordnete Magnete (700) zwischen der ersten Stelle und der zweiten Stelle positioniert sind.

5. Tragbare Rechenvorrichtung nach einem der vorhergehenden Ansprüche, wobei eine Breite des Spalts zwischen der Leiterplatte und dem leitfähigen Gehäuse im Bereich von etwa 0,5 Millimetern bis etwa 10 Millimetern liegt.

6. Tragbare Rechenvorrichtung nach Anspruch 1, wobei ein Umfang der Leiterplatte einen massefreien Bereich (306) umfasst.

7. Tragbare Rechenvorrichtung nach Anspruch 6, wobei eine Breite der Schlitzantenne die Breite des Spalts und eine Breite des massefreien Bereichs überspannt.

8. Tragbare Rechenvorrichtung nach Anspruch 7, wobei die Breite der Schlitzantenne im Bereich von etwa 0,5 Millimetern bis etwa 10 Millimetern liegt.

9. Tragbare Rechenvorrichtung nach einem der vorhergehenden Ansprüche, welche so konfiguriert ist, dass, wenn die Schlitzantenne in dem einen oder den mehreren GPS-Frequenzbändern arbeitet, die Schlitzantenne einen oder mehrere elektrische Ströme auf dem parasitären Element induziert.

10. Tragbare Rechenvorrichtung nach einem der vorhergehenden Ansprüche, wobei die Schlitzantenne einen ersten Massekontakt (502) und einen zweiten Massekontakt (504) umfasst, wobei der erste Massekontakt an einer ersten Stelle zwischen der Leiterplatte und dem leitfähigen Gehäuse gekoppelt ist, und der zweite Massekontakt an einer zweiten Stelle, die sich von der ersten Stelle unterscheidet, zwischen der Leiterplatte und dem leitfähigen Gehäuse gekoppelt ist.

11. Tragbare Rechenvorrichtung nach einem der vorhergehenden Ansprüche, wobei das eine oder die mehreren GPS-Frequenzbänder umfassen:
ein erstes GPS-Frequenzband im Bereich von etwa 1164 MHz bis etwa 1189 MHz
ein zweites GPS-Frequenzband im Bereich von etwa 1563 MHz bis etwa 1587 MHz; und
ein drittes GPS-Frequenzband im Bereich von etwa 1215 MHz bis etwa 1240 MHz.

12. Tragbare Rechenvorrichtung nach Anspruch 2, welche so konfiguriert ist, dass eine Strahlungseffizienz der Schlitzantenne in dem einen oder den mehreren GPS-Frequenzbändern um mindestens 2 Dezibel erhöht wird, was zumindest teilweise darauf zurückzuführen ist, dass das parasitäre Element über den einen oder die mehreren Überbrückungskondensatoren für HF mit der Leiterplatte an Masse gelegt ist.

13. Tragbare Rechenvorrichtung nach einem der vorhergehenden Ansprüche, wobei:
das parasitäre Element an einer ersten Stelle darauf über eine erste Federklammer für HF mit der Leiterplatte an Masse gelegt ist; und
das parasitäre Element an einer zweiten Stelle darauf über eine zweite Federklammer für HF mit der Leiterplatte an Masse gelegt ist.

14. Tragbare Rechenvorrichtung nach einem der vorhergehenden Ansprüche, ferner umfassend:
eine Elektrode für elektrodermale Aktivität, die elektrisch mit der Leiterplatte gekoppelt ist.

## Revendications

1. Dispositif informatique portable (100), comprenant :
une carte de circuit imprimé (300) ;
un boîtier conducteur (120) ;
une antenne à fentes (500) définie par un espace (400) entre la carte de circuit imprimé et le boîtier conducteur, l'antenne à fentes fonctionnant sur une pluralité de bandes de fréquences différentes, la pluralité de bandes de fréquences différentes comprenant une ou plusieurs bandes de fréquences d'un système de positionnement global, GPS ; et
un élément parasite (600), l'élément parasite étant mis à la terre électriquement sur la carte de circuit imprimé à plusieurs emplacements différents, le dispositif informatique portable étant **caractérisé en ce que**
l'élément parasite est une électrode d'électrocardiogramme, ECG, électrode (200).

2. Dispositif informatique portable selon la revendication 1, dans lequel l'élément parasite est une radiofréquence, RF, mise à la terre sur la carte de circuit imprimé via un ou plusieurs condensateurs de dérivation (610, 612).

3. Dispositif informatique portable selon la revendication 1, dans lequel :
l'élément parasite est un courant continu, CC, mis à la terre sur la carte de circuit imprimé à un premier emplacement (602) sur celle-ci ; et
l'élément parasite est un CC, mis à la terre sur la carte de circuit imprimé à un second emplacement (604) sur celle-ci.

4. Dispositif informatique portable selon la revendication 3, dans lequel le premier emplacement et le second emplacement sont espacés l'un de l'autre sur la carte de circuit imprimé de telle sorte qu'un ou plusieurs aimants (700) disposés sur la carte de circuit imprimé soient positionnés entre le premier emplacement et le second emplacement.

5. Dispositif informatique portable selon l'une quelconque des revendications précédentes, dans lequel une largeur de l'espace entre la carte de circuit imprimé et le boîtier conducteur varie d'environ 0,5 millimètre à environ 10 millimètres.

6. Dispositif informatique portable selon la revendication 1, dans lequel un périmètre de la carte de circuit imprimé comprend une zone d'exclusion de la masse (306).

7. Dispositif informatique portable selon la revendication 6, dans lequel une largeur de l'antenne à fentes couvre la largeur de l'espace et une largeur de la zone d'exclusion de la masse.

8. Dispositif informatique portable selon la revendication 7, dans lequel la largeur de l'antenne à fentes varie d'environ 0,5 millimètre à environ 10 millimètres.

9. Dispositif informatique portable selon l'une quelconque des revendications précédentes, configuré de telle sorte que, lorsque l'antenne à fentes fonctionne sur les une ou plusieurs bandes de fréquences de GPS, l'antenne à fentes induit un ou plusieurs courants électriques sur l'élément parasite.

10. Dispositif informatique portable selon l'une quelconque des revendications précédentes, dans lequel l'antenne à fentes comprend un premier contact de mise à la terre (502) et un second contact de mise à la terre (504), le premier contact de mise à la terre étant couplé entre la carte de circuit imprimé et le boîtier conducteur à un premier emplacement, le second contact de mise à la terre étant couplé entre la carte de circuit imprimé et le boîtier conducteur à un second emplacement différent du premier emplacement.

11. Dispositif informatique portable selon l'une quelconque des revendications précédentes, dans lequel les une ou plusieurs bandes de fréquences de GPS comprennent :
une première bande de fréquences de GPS allant d'environ 1164 MHz à environ 1189 MHz
une deuxième bande de fréquences de GPS allant d'environ 1563 MHz à environ 1587 MHz ; et
une troisième bande de fréquences de GPS allant d'environ 1215 MHz à environ 1240 MHz.

12. Dispositif informatique portable selon la revendication 2, configuré de telle sorte qu'une efficacité de rayonnement de l'antenne à fentes sur les une ou plusieurs bandes de fréquences de GPS soit augmentée d'au moins 2 décibels grâce, au moins en partie, à la mise à la terre RF de l'élément parasite sur la carte de circuit imprimé via les un ou plusieurs condensateurs de dérivation.

13. Dispositif informatique portable selon l'une quelconque des revendications précédentes, dans lequel :
l'élément parasite est mis à la terre RF sur la carte de circuit imprimé à un premier emplacement via une première une première pince à ressort ; et
l'élément parasite est mis à la terre RF sur la carte de circuit imprimé à un second emplacement via une seconde pince à ressort.

14. Dispositif informatique portable selon l'une quelconque des revendications précédentes, comprenant également :
une électrode d'activité électrodermale couplée électriquement à la carte de circuit imprimé.
